# EUROPÄISCHE PATENTSCHRIFT

(11) **EP 0 426 098 B1**
(45) Veröffentlichungstag und Bekanntmachung des Hinweises auf die Patenterteilung: **08.06.1994**
(21) Anmeldenummer: 90120778.7
(22) Anmeldetag: 30.10.1990
(51) Int. Cl.: A61K 31/19, A61K 33/06, A61K 33/08, A61K 33/10

(54) **Phosphatbinder zur oralen Verabreichung**
Phosphate binding agent for oral administration
Agent liant de phosphate pour l'administration orale

(30) Priorität: 01.11.1989 DE 3936319
(43) Veröffentlichungstag der Anmeldung: 08.05.1991
(73) Patentinhaber: Bartz, Volker, 35625 Hüttenberg (DE)
(72) Erfinder: Gretz, Norbert, Dr., W-6800 Mannheim 51 (DE); Zimmermann, Eckhard, Dr., W-6800 Mannheim 1 (DE)
(74) Vertreter: Barz, Peter, Dr.

(56) Entgegenhaltungen:
- FR-M- 827
- PATENT ABSTRACTS OF JAPAN, vol. 10, no. 194, (C-358)(2250), 08 Juli 1986#

## Beschreibung

Die Erfindung betrifft die Herstellung eines Arzneimittels für die Behandlung des hyperphosphatämischen Status.

Die konservative Therapie der chronischen Niereninsuffizienz hat, trotz der in den letzten Jahren beeindruckenden Ergebnisse durch Dialyse und Transplantation, speziell in den prädialytischen Stadien weiterhin eine entscheidende Bedeutung im Hinblick auf das Langzeitergebnis. Diese konservative Therapie umfaßt die Behandlung des zu Grunde liegenden Leidens, die Diätetik und symptomatisch bedingte medikamentöse Maßnahmen.

Neben metabolischer Azidose, renaler Anämie, Störungen im kardiovaskulären-, gastrointestinalen- und Skelett-System, darüberhinaus Störungen im intermediären Stoffwechsel, weisen Patienten mit chronischer Niereninsuffizienz und Dialysepatienten im allgemeinen pathologisch erhöhte Serum-Phosphat-Konzentrationen (über 1,45 mmol/l) auf. Die Entwicklung einer solchen Hyperphosphatämie ist verbunden mit der Abnahme des Glomerulusfiltrats. Diese sind ein ursächlicher Faktor für den sekundären Hyperparathyreoidismus, der als Teilursache der renalen Osteopathie betrachtet wird.

Die Behandlung des hyperphosphatämischen Status, das heißt die Senkung des Serumphosphats, um eine Suppression des Hyperparathyreoidismus zu erzielen, ist bislang noch unbefriedigend gelöst. Eine Übersicht zu diesem Thema findet sich in der wissenschaftlichen Literatur in: Schaefer, K., von Herrath, D., Erley, C.M.: Aktuelle Aspekte in der Therapie der urämischen Hyperphosphatämie. Dtsch. med. Wschr. 112, Nr. 44, (1987), 1708-1712.
Üblicherweise werden zur Senkung des Serumphosphats, neben einer Diätverordnung, medikamentös oral verabreichte Phosphatbinder eingesetzt, die meist aluminiumhaltig sind. So werden zum Beispiel der phosphatbindenden Eigenschaften von Aluminiumhydroxid bei nierenkranken Patienten routinemässig therapeutisch genutzt. Die tägliche Dosis liegt üblicherweise bei ca. 4 g Aluminiumhydroxid. Dabei wird nicht nur in der Nahrung zugeführtes Phosphat durch das im Magen in Gegenwart von Magensäure ( Salzsäure ) gebildete Aluminiumchlorid in unlösliches Aluminiumphosphat übergeführt, sondern zusätzlich wird aus dem Blut in das Darmlumen sezerniertes Phosphat gebunden. Das somit in eine schwerlösliche Form überführte Phosphat ( = Aluminium-phosphat ) wird mit den Fäces ausgeschieden.

Es ist allgemein wissenschaftlich anerkannt, daß Aluminium das Dialyse-Enzephalopathie-Syndrom verursacht. Es ist verantwortlich für die osteomalazische Komponente der renalen Osteodystrophie und kann zusätzlich zur Verstärkung der renalen Anämie beitragen. Nachdem anfänglich lediglich mit Aluminium belastetes Dialysat als Aluminiumquelle bei chronisch niereninsuffizienten Hämodialyse-Patienten in Betracht gezogen wurde, zeigte sich nach dessen hochgradiger Reinigung mit Hilfe von Umkehrosmoseverfahren, daß diese Patienten beachtliche Mengen aluminiumhaltiger Phosphatbinder aufnehmen, die als nicht resorbierbar galten.

Um das Risiko dieser inzwischen gut bekannten Aluminium-Intoxikation bei der oralen Applikation aluminiumhaltiger Phosphatbinder zu umgehen, wurden daraufhin Magnesiumsalze und Calciumsalze als Phosphatbinder gastrointestinal eingesetzt. Das Wirkprinzip dieser divalenten Metallsalze entspricht dem des Aluminium.

Magnesiumhaltige Präparate wirken laxativ und verursachen deshalb oft Durchfälle, im Gegensatz zu obstipierend wirkenden Aluminiumpräparaten. Insbesondere bei eingeschränkter Nierenfunktion gilt für Magnesium das gleiche wie für Aluminium, das heißt, die Akkumulation und Intoxikation bei chronischer Zufuhr (hier: Hypermagnesiämie).

Die Verwendung calciumhaltiger Präparate erfolgt meist in Form des Calciumcarbonats mit einer täglichen Dosis von ca. 6 g, sinnvollerweise eingenommen zu den Mahlzeiten, um genügend Phosphat zu binden. Hierbei werden, insbesondere bei zusätzlicher Vitamin-D3 Gabe häufig Hypercalciämien induziert ( Gefahr der Nephrocalcinose ). Infolge hoher Serumcalciumwerte ist die Ausschüttung von Parathormon vermindert, so daß es zu einem unerwünschten Anstieg des Serumphophats kommen kann.

Neben Calciumcarbonat sind in der Literatur weitere Calciumverbindungen als gastrointestinale Phosphatbinder beschrieben worden, wie zum Beispiel Calciumalginat, Calciumgluconat und Calciumcitrat. In der Praxis haben diese Verbindungen allerdings keine Bedeutung erlangt, da die phosphatbindenden Kapazitäten zu gering waren, beziehungsweise unerwünschte Nebeneffekte, wie zum Beispiel die resorptionsfördernde Wirkung von Citrat auf Metall-Ionen, auftraten.

Bei der diätetischen Behandlung von chronisch niereninsuffizienten Patienten mit dem Ziel, den gestörten Aminosäurenhaushalt zu regulieren und eine zusätzliche Stickstoffbelastung des Organismus zu vermeiden, beziehungsweise eine Stickstoffreutilisation zu erzielen, werden Gemische von alpha-Ketoanalogen essentieller Aminosäuren mit Erfolg, oral eingenommen, eingesetzt, wie beispielsweise in den Druckschriften DE 25 31 299 A1, DE 30 15 076 C2, DE-OS 23 35 215, US 44 91 589 A, WO 87 03 806 A1 oder DE 30 46 580 A1 beschrieben. Solche Gemische können auch Ketoanaloge nicht essentieller Aminosäuren enthalten. Durch Transaminierung werden aus diesen Ketosäuren endogen essentielle bzw. nichtessentielle Aminosäuren gebildet. Als Nebeneffekt solcher Therapien zeigte sich, daß die Calciumsalze dieser Ketoanalogen der essentiellen Aminosäuren eine Erniedrigung des Serumphosphats bewirkten, die allerdings auch bei sehr hoher Dosierung (bis 10 g / Tag) nicht ausreichend sind, um auf herkömmliche Phosphatbinder zu verzichten. Insbesondere bekannt ist diese Eigenschaft bei Gemischen der Calciumsalze der Ketoanalogen der verzweigtkettigen essentiellen Aminosäuren Valin, Leucin und Isoleucin. Weiterhin konnte nachgewiesen werden, daß die gleiche Konzentration von Calciumionen aus diesen alpha-Ketoanalogen Verbindungen in der Kapazität der Phosphatelimination höher ist als bei Calciumcarbonat. Diese Effekte wurden bisher allerdings nur mit Gemischen von Ketoanalogen essentieller Aminosäuren beschrieben, wie beispielsweise von Schaefer et. al. (Schaefer, K., Herrath, D., Asmus, G., Umlauf, E.: The beneficial effect of ketoacids on serum phosphate and parathyroid hormone in patients with chronic uremia. Clin. Nephrol. 30 (2), 93, 1988), nicht jedoch mit Ketoanalogen nicht-essentieller Aminosäuren.

Weitere therapeutische Einsatzmöglichkeiten finden Ketoanaloge nicht-essentieller Aminosäuren zur Auflösung, beziehungsweise Prophylaxe, von Harnsteinen (vgl. DE 30 46 580 A1).

Nachteilig beim Einsatz von alpha-Ketoanalogen von essentiellen Aminosäuren zu bewerten ist das Risiko der Induktion von Aminosäurenimbalanzen, welche zu schweren Stoffwechselanomalien oder katabolen Zuständen führen. Solche Aminosäurenimbalanzen bedeuten, daß bestimmte einzelne essentielle Aminosäuren in ihrer Konzentration stark erhöht oder erniedrigt vorliegen, also das zum Proteinaufbau definierte Verhältniss essentieller Aminosäuren untereinander ( Aminosäurenmuster ) gestört ist. In diesem Zusammenhang spricht man von sogenannten "limitierten Aminosäuren". Das Vermeiden solcher Aminosäurenimbalanzen bei der exogenen Zufuhr von essentiellen Aminosäuren oder alpha-Ketoanalogen essentieller Aminosäuren ist somit nur dann möglich, wenn das Verhältnis aller essentiellen Aminosaüren in physiologischen Grenzen gehalten wird. In der Praxis bedeutet das, daß bei der chronischen Supplementation einer essentiellen Aminosäure oder eines alpha-Ketoanalogen einer essentiellen Aminosäure alle anderen essentiellen Aminosäuren oder alpha-Ketoanaloge dieser essentiellen Aminosäuren in ausreichender Konzentration zugeführt werden müssen, um Stoffwechselentgleisungen zu vermeiden. Dies hat zur Folge, daß Ketoanaloge essentieller Aminosäuren immer nur in Kombinationspräparaten langfristig eingesetzt werden können und als solche auch auf dem pharmazeutischen Markt angeboten werden. Ein weiterer damit verbundener Nachteil besteht in den hohen Kosten essentieller Aminosäuren, beziehungsweise ihrer Ketoanalogen, insbesondere der unabdinglich in diesen Präparaten notwendigen essentiellen verzweigtkettigen Aminosäuren.

Es war auch bekannt (FR-M-827), Calcium-alpha-Ketoglutarat als Medikament zur Behandlung von Geistesschwäche oder zur Entgiftung von Stickstoffprodukten zu verwenden, insbesondere bei Hyperazotemie, Lebervergiftungen durch Stickstoff-Medikamente, Funktionsstörungen der Leber bei der organischen Umsetzung von Stickstoffmineralien, Müdigkeitszuständen durch Ansammlung von Ammoniakderivaten. Die Dosierung pro Tag sollte dabei 2 - 4 g alpha-Ketoglutarat in Form von Tabletten, Granulat, trinkfertigen oder injizierbaren Lösungen betragen.

Die der Erfindung zugrundeliegende Aufgabe ist es hingegen, ein Arzneimittel für die Behandlung des hyperphosphatämischen Status herzustellen. Der nach diesem Verfahren hergestellte Phosphatbinder soll über längere Zeit an Patienten mit Hyperphosphatämie oral applizierbar sein, ohne daß unerwünschte Nebenwirkungen auftreten, wobei Aluminium- und Magnesiumintoxikationen vermieden und Hypercalcämien und Stoffwechselentgleisungen vermindert werden sollen und eine ausreichende Phosphatelimination gewährleistet ist.
Darüberhinaus soll ein positiver Beitrag zur Verbesserung des Intermediärstoffwechsels sichergestellt werden.

Diese Aufgabe wird durch die Verwendung von alpha-Ketoglutarsäure-Calciumsalz zur Herstellung des Arzneimittels für die Behandlung des hyperphosphatämischen Status gelöst.

Es wurde festgestellt, daß die danach hergestellten Arzneimittel hohe phosphatbindende Eigenschaften aufweisen und über längere Zeit verordnet werden können, ohne daß die bei den bisher üblichen Phosphatbindern üblichen Nebenwirkungen auftreten.

Im einzelnen werden diese Vorteile erzielt, wenn bei der Herstellung des Arzneimittels ein Elektrolytgemisch nach Anspruch 2 hergestellt wird.

Es hat sich gezeigt, daß in dem danach hergestellten Arzneimittel das Calciumsalz der alpha-Ketoglutarsäure oral appliziert genügend hohe phosphatbindende Eigenschaften besitzt, um langfristig als alleiniger Phosphatbinder in einer Konzentration von 1 bis 5 Gramm pro Tag bei hyperphosphatämischen Patienten ausreichende Serumphosphatsenkungen (d.h. Vermeidung einer Hyperphosphatämie) prädialytisch zu bewirken.

Besonders vorteilhafte Quantifizierungen der Herstellung sind in Anspruch 3 angegeben.

Unerwartet trat eine Serumphosphatsenkung, wenn auch nicht so ausgeprägt wie bei den nach den Ansprüchen 1-3 hergestellten Arzneimitteln, auch bei solchen Arzneimitteln auf, für deren Herstellung nach Anspruch 4 ein anderes mono-, di- oder trivalentes Metallsalz der alpha-Ketoglutarsäure verwendet wurde, insbesondere alpha-Ketoglutarsäure-Natriumsalz. Der diesbezügliche Wirkungsmechanismus ist bisher unklar, dürfte aber auf metabolische Effekte (Katabolie, Proteinbiosynthese) zurückzuführen sein.
Die phosphatbindende Eigenschaft von alpha-Ketoglutarsäure-Calciumsalz konnte auch in vitro (Methode nach: Sheikh, M.S., Maguire, J.A., Emmett, M., Santa Ana, C.A., Nicar, M.J., Schiller, L.R., Fordtran, J.S.: Reduction of dietary phosphorus absorption by phosphorus binders - A theoretical, in vitro, and in vivo study. J. Clin. Invest. 83, 66-73, 1989) nachgewiesen werden. Alpha-Ketoglutarsäure-Calciumsalz bindet im äquimolaren Vergleich zu dem herkömmlichen Phosphatbinder Calciumcarbonat im physiologischen pH-Bereich des Darmes (pH 7,4) etwa 3-mal mehr Phosphat.

Bei der erfindungsgemäßen Anwendung von alpha-Ketoglutarsäure-Calciumsalz-haltigen Phosphatbindern ergeben sich folgende Vorteile:
Die alpha-Ketoglutarsäure ( = 2-Oxo-Glutarsäure ) ist das Ketoanaloge der nicht-essentiellen Aminosäure Glutamat, die in der Lebensmittelindustrie in großem Umfang als Geschmacksverstärker, zum Beispiel in Gewürzmischungen, Verwendung findet. Somit sind auch die Molekulargewichte (MG) der beiden festen Substanzen alpha-Ketoglutarsäure (MG=146) und Glutaminsäure (MG=147) nahezu identisch. Die technische Handhabung beider Substanzen sind hinlänglich bekannt und entsprechen sich in ihrer Einfachheit.

Alpha-Ketoglutarsäure ist ein leicht erhältliches Handelsprodukt. Im Handel ist hochreine alpha-Ketoglutarsäure mit einem Reinheitsgrad von mehr als 99 % erhältlich.

Durch einfaches äquimolares Mischen und in wäßrige Lösung bringen von reiner alpha-Ketoglutarsäure und einem Calciumsalz, wie zum Beispiel Calciumcarbonat, läßt sich technisch leicht ein alpha-Ketoglutarsäure-Calciumsalz herstellen. Bei dieser chemischen Reaktion zwischen alpha-Ketoglutarsäure und Calciumcarbonat (Kalkspat, Kreide, Marmor) entsteht alpha-Ketoglutarsäure-Calciumsalz und Kohlensäure, welche als Kohlendioxid entweicht.

Im Gastrointestinaltrakt dissoziiert Calcium aus der alpha-Ketosäurenverbindung, und zwar weitaus besser, als zum Beispiel aus Carbonatverbindungen, und bindet dadurch effektiver Phosphat. Dies bedeutet, daß die notwendige absolut zugeführte Menge an Calciumionen zur Phosphatbindung bei alpha-Ketoglutarsäure-Calciumsalz geringer ist, als bei Calciumcarbonat. Hypercalcämien werden vermieden.

Im Gegensatz zu Magnesiumsalzen wird alpha-Ketoglutarsäure vollständig enteral resorbiert. Die durch Magnesium, als Phosphatbinder verabfolgt, hervorgerufenen Durchfälle werden, ebenso selbstverständlicherweise Hypermagnesiämien, durch die Verabreichung von alpha-Ketoglutarsäure-Calciumsalz verhindert.

Die Marktpreise für alpha-Ketoglutarat sind weitaus günstiger als die der Ketosäuren essentieller Aminosäuren, insbesondere der verzweigtkettigen Aminosäuren. Daraus ergibt sich ein beträchtlicher ökonomischer Vorteil.

Die physiologischen Eigenschaften der alpha-Ketoglutarsäure im intermediären Stoffwechsel sind hinreichend bekannt. Im Citratzyklus ( = Krebszyklus nach H. A. Krebs, Oxford, Nobelpreis 1954 ), in den der Kohlenhydrat-, Eiweiß- bzw. Aminosäuren- und Fettstoffwechsel in Form der aktivierten Essigsäure einmünden, nimmt alpha-Ketoglutarsäure eine dominierende Schlüsselstellung ein, indem der intermediäre Stoffwechsel dieser Nährstoffe somit über alpha-Ketoglutarsäure als Zwischenprodukt abläuft. Durch diese physiologische Stellung von alpha-Ketoglutarsäure im Intermediärstoffwechsel ist die pharmakologisch toxikologische Unbedenklichkeit gewährleistet.

Insbesondere akkumuliert alpha-Ketoglutarat nicht in Geweben, im Gegensatz zu Aluminium bei der Verabreichung aluminiumhaltiger Phosphatbinder.

Stoffwechselentgleisungen in Form von Aminosäurenimbalanzen sind bei einer vermehrten Zufuhr von alpha-Ketoglutarat, im Gegensatz zu Ketoanalogen essentieller Aminosäuren, nicht zu erwarten, da die analoge Aminosäure Glutamat, die durch endogene Transaminierung gebildet werden kann, eine nichtessentielle Aminosäure ist und somit die Proteinsynthese nicht limitiert. Durch diese Transaminierungsreaktion wird, wie bei der Verabreichung von Ketoanalogen essentieller Aminosäuren therapeutisch genutzt, endogen Stickstoff gebunden. Dies ist insbesondere bei der bei Niereninsuffizienz üblicherweise notwendigen proteinarmen Diät im Rahmen einer Stickstoffreutilisation von Vorteil.

Der Abbau der, bei chronischer Niereninsuffizienz in zu geringer Konzentration gefundenen, essentiellen verzweigtkettigen Aminosäuren Valin, Leucin und Isoleucin erfolgt biochemisch im ersten, reversiblen Schritt mit Hilfe unspezifischer Isoenzyme (Transaminasen) zur entsprechenden alpha-Ketosäure dieser Aminosäuren. Der nachfolgende Reaktionsschritt, die dehydrierende Decarboxylierung zum Fettsäure-Coenzym-A-Thioester ist nicht reversibel. Bei entsprechend hohen Konzentrationen von alpha-Ketosäuren erfolgt eine in der Biochemie übliche sogenannte Produkthemmung des Abbaus, das heißt, die durch den Abbau verzweigtkettiger Aminosäuren entstehenden alpha-Ketosäuren (Produkte) hemmen entsprechend ihrer Konzentration diesen Abbau. Da diese Produkthemmung im Falle des Abbaus verzweigtkettiger Aminosäuren zu alpha-Ketosäuren unspezifisch hinsichtlich der alpha-Ketosäure ist, wird eine solche Produkthemmung auch von alpha-Ketoglutarat ausgehen. Dadurch werden die essentiellen verzweigtkettigen Aminosäuren vor dem Abbau geschützt.

Somit ergeben sich bei der neuen erfindungsgemäßen Verwendung von alpha-Ketoglutarsäure-Calciumsalz zur oralen Verabreichung als Phosphatbinder anstatt der bisher verwendeten Phosphatbinder wie Aluminiumverbindungen, Magnesiumsalzen, Calciumcarbonat, Calciumalginat, Calciumcitrat, Calciumgluconat oder Ketoanalogen essentieller Aminosäuren alle diesbezüglich dieser bisherigen Phosphatbinder bekannten positiven Eigenschaften ohne deren Nachteile.
Der erfindungsgemäß verwendete Phosphatbinder besteht aus einem Metallsalz der alpha-Ketoglutarsäure, vorzüglich aus alpha-Ketoglutarsäure-Calciumsalz. Die Herstellung kann nach den bekannten Verfahren zur Herstellung phosphatbindender Substanzen erfolgen, insbesondere durch äquimolares Mischen von Calciumcarbonat und alpha-Ketoglutarsäure und nachfolgender wässriger Lösung, wodurch alpha-Ketoglutarsäure-Calciumsalz (alpha-Keto-Calciumglutarat) als phosphatbindende Substanz und Kohlensäure, welche als Kohlendioxid entweicht, entstehen.
Wenn der erfindungsgemäß verwendete Phosphatbinder neben alpha-Ketoglutarsäure auch Calciumcarbonat als weiteren fakultativen Phosphatbinder enthält, so beträgt das Mengenverhältnis vorteilhafterweise 1:3 bis 3:1, vorzugsweise 1:1. Dieser Phosphatbinder kann aber auch gewünschtenfalls nicht nur Calciumcarbonat, sondern auch z. B. calciumhaltige nicht carbonathaltige Substanzen, aluminium- oder magnesiumhaltige Substanzen, Ketoanaloge / Aminosäuren und deren Salze, sowie Peptide als weitere, fakultativ phosphatbindende Substanzen enthalten.
Der erfindungsgemäß verwendete Phosphatbinder kann neben alpha-Ketoglutarsäure-Calciumsalz auch andere mono-, di- oder trivalente Metallsalze der alpha-Ketoglutarsäure, wie z. B. Natrium-, Kalium- oder Magnesiumsalze, sowie andere Substanzen enthalten, wie z. B. Wasser, Alkohole, Kohlenhydrate, organische und anorganische Säuren, Fette, Proteine, Aromastoffe, Mineralstoffe und Spurenelemente und andere in der pharmazeutischen Industrie bekannte Zusatzstoffe und Hilfsstoffe.

Die zur Anwendung kommenden alpha-Ketoglutarat-Calciumsalz-Konzentrationen liegen im Bereich von 0,1 bis 30 Gramm als phosphatbindende Substanz zur oralen Verabreichung pro Einzeldosis. Vorteilhafterweise liegen sie im Bereich von 0,5 bis 20 Gramm, insbesondere 1 bis 5 Gramm. Um eine effektive Phosphatbindung und -elimination zu erzielen, wird günstigerweise jeweils eine Einzeldosis zu jeder Mahlzeit eingenommen.

Die Darreichungsform des erfindungsgemäß verwendeten Phosphatbinders kann in jeder bekannten Form zur oralen Verabfolgung einer Substanz erfolgen, sofern eine gastrointestinale Dissoziation der Substanz in der applizierten Form möglich ist, wie z. B. in fester Form als Tablette, Dragee, Kapsel, Pulver, Granulat oder in halbfester Form als Gel, Emulsion, Suspension oder in flüssiger Form.

### Beispiel 1:

Es werden 1 Mol Calciumcarbonat (Pulver) und 1 Mol alpha-Ketoglutarsäure (Pulver) gemischt. Von dieser Mischung werden zwei Gramm ad 100 ml Wasser aufgelöst. Es entweicht Kohlendioxid und entsteht eine wässrige Lösung aus alpha-Ketoglutarsäure-Calciumsalz.

| | |
|---|---|
| alpha Ketoglutarsäure Calciumsalz | 2 g/100 ml |

### Beispiel 2:

Beispiel 1 wurde wiederholt, jedoch mit der Ausnahme, daß ein Gramm ad 100 ml aufgelöst wurden.

| | |
|---|---|
| alpha-Ketoglutarsäure-Calciumsalz | 1 g/100 ml |

## Patentansprüche

1. Verwendung von alpha-Ketoglutarsäure-Calciumsalz zur Herstellung eine Arzneimittels für die Behandlung des hyperphosphatämischen Status.

2. Verwendung von alpha-Ketoglutarsäure-Calciumsalz zur Herstellung eines Arzneimittels nach Anspruch 1, wobei ein Elektrolytgemisch hergestellt wird, welches pro Einzeldosis eine Menge von 0,1 bis 30 Gramm an alpha-Ketoglutarsäure-Calciumsalz enthält.

3. Verwendung von alpha-Ketoglutarsäure-Calciumsalz zur Herstellung eines Arzneimittels nach Anspruch 1, wobei ein Elektrolytgemisch hergestellt wird, welches pro Einzeldosis eine Menge von 0,2 bis 10 Gramm an alpha-Ketoglutarsäure-Calciumsalz, vorzugsweise 0,5 bis 2 Gramm, enthält.

4. Verwendung eines anderen mono-, di- oder trivalenten Metallsalzes der alpha-Ketoglutarsäure anstelle des alpha-Ketoglutarsäure-Calciumsalzes zur Herstellung eines oral verabreichbaren Phosphatbinders für die Behandlung von Hyperphosphatämie, wobei ein Elektrolytgemisch hergestellt wird, welches das Metallsalz der alpha-Ketoglutarsäure enthält.

5. Verwendung von alpha-Ketoglutarsäure-Calciumsalz oder eines anderen mono-, di- oder trivalenten Metallsalzes der alpha-Ketoglutarsäure zur Herstellung eines oral verabreichbaren Phosphatbinders zur Behandlung von Hyperphosphatämie, wobei ein Elektrolytgemisch hergestellt wird, welches pro Einzeldosis eine Menge von 0,2 bis 10 Gramm an alpha-Ketoglutarsäure-Calciumsalz, vorzugsweise 0,5 bis 2 Gramm, enthält.

## Claims

1. Use of calcium α-ketoglutarate in producing a pharmaceutical agent for treating the hyperphosphoremic state.

2. Use of calcium α-ketoglutarate in producing a pharmaceutical agent according to claim 1, whereby an electrolyte mixture is prepared that contains an amount of 0.1 to 30 grams of calcium α-ketoglutarate per single dose.

3. Use of calcium α-ketoglutarate in producing a pharmaceutical agent according to claim 1, whereby an electrolyte mixture is prepared that contains an amount of 0.2 to 10 grams of calcium α-ketoglutarate, preferably 0.5 to 2 grams, per single dose.

4. Use of another mono-, di-, or tri-valent metal salt of α-ketoglutaric acid, instead of calcium α-ketoglutarate, in producing an orally administered phosphate binder for treating hyperphosphoremia, whereby an electrolyte mixture is prepared that contains the metal salt of the α-ketoglutaric acid.

5. Use of calcium α-ketoglutarate or another mono-, di-, or tri-valent metal salt of α-ketoglutaric acid in producing an orally administered phosphate binder for treating hyperphosphoremia, whereby an electrolyte mixture is prepared that contains an amount of 0.2 to 10 grams of calcium α-ketoglutarate, preferably 0.5 to 2 grams, per single dose.

## Revendications

1. Utilisation du sel de calcium de l'acide alpha-cétoglutarique pour préparer un médicament pour le traitement d'un état d'hyperphosphatémie.

2. Utilisation du sel de calcium de l'acide alpha-cétoglutarique pour préparer un médicament selon la revendication 1, selon laquelle on prépare un mélange d'électrolytes, qui contient, par dose unitaire, une quantité de 0,1 à 30 g du sel de calcium de l'acide alpha-cétoglutarique.

3. Utilisation du sel de calcium de l'acide alpha-cétoglutarique pour préparer un médicament selon la revendication 1, selon laquelle on prépare un mélange d'électrolytes, qui contient par dose unitaire une quantité de 0,2 à 10 g, avantageusement 0,5 à 2 g, de sel de calcium de l'acide alpha-cétoglutarique.

4. Utilisation d'un autre sel de métal monovalent, divalent ou trivalent de l'acide alpha-cétoglutarique au lieu du sel de calcium de l'acide alpha-cétoglutarique pour préparer un liant de phosphate administrable par voie orale pour le traitement d'une hyperphosphatémie, selon laquelle on prépare un mélange d'électrolytes qui contient le sel de métal de l'acide alpha-cétoglutarique.

5. Utilisation du sel de calcium de l'acide alpha-cétoglutarique ou d'un autre sel de métal monovalent, divalent ou trivalent de l'acide alpha-cétoglutarique pour préparer un liant de phosphate, administrable par voie orale, pour traiter une hyperphosphatémie, selon laquelle on prépare un mélange d'électrolytes qui contient, par dose unitaire, une quantité de 0,2 à 10 g d'un sel de calcium de l'acide alpha-cétoglutarique, avantageusment 0,5 à 2 g.
